# DEMANDE DE BREVET EUROPEEN

(11) **EP 1 033 110 A2**
(43) Date de publication de la demande: **06.09.2000**
(21) Numéro de dépôt: 00500001.3
(22) Date de dépôt: 10.01.2000
(51) Int. Cl.: A61B 17/54

(54) **Dispositif pour le soin et le traitement de cors et de durillons**

(30) Priorité: 13.01.1999 ES 9900074 U
(71) Demandeur: Pascual Gombau, Amado, 08211 Castellar del Valles (ES)
(72) Inventeur: Pascual Gombau, Amado, 08211 Castellar del Valles (ES)
(74) Mandataire: Espiell Volart, Eduardo Maria

(57) **Abrégé**

Dispositif pour le soin et le traitement de cors et de durillons constitué par un manche de base à l'extrémité duquel est agencée une pièce métallique comme cuiller-support pourvue du dispositif basculant de tenue et de fixation de la lame utilisée pour l'opération de coupage, caractérisé en ce que cette cuiller-support (2) dispose de deux fenêtres (4) (4') rectangulaires, parallèles entre elles et perpendiculaires à l'axe de l'ensemble, utilisées pour fixer la lame (3) et à travers desquelles apparaissent ses bords tranchants, tout cela de sorte qu'on pourra y placer et fixer à volonté une lame complémentaire (7), métallique et élastique, à forme courbe qui sera fixée par flexion dans ces fenêtres (4) (4'), la convexité de cette lame restant vers l'extérieur. Cette lame complémentaire (7), possède une série de grains (8), lesquels occupent toute la surface externe, formés par emboutissage et complétés par découpage partiel à l'emporte-pièce, de sorte que tous présentent des arêtes d'extrémité tranchantes, pour exercer la fonction de raclage requise.

## Description

La présente invention concerne un dispositif destiné au soin et au traitement de cors et de durillons offrant diverses innovations et avantages sur ce qui existe actuellement et utilisé dans le même but.

On connaît les ustensiles permettant le soin notamment et précisément des pieds, en coupant et en éliminant les callosités et les durillons qui sont produits par l'effet de la pression de la chaussure, en général, sur certains points saillants du pied, et constitués par des couches de tissu épithélial, de cellules mortes.

Plusieurs de ceux-ci sont basés sur la possibilité de pouvoir agir directement sur ces irrégularités grâce à l'agencement spécial d'une lame appropriée sur un support, permettant d'effectuer la coupe des zones en saillie sans risquer de blesser par coupage incisif accidentel.

Ces ustensiles sont limités dans leur action car à certains moments il ne suffit pas d'utiliser une lame tranchante ou bien ça n'est pas possible et il ne convient pas de le faire et il faut procéder à rabaisser en raclant avec une surface rugueuse, telle qu'une lime. Il faut pour cela utiliser plus d'un ustensile, ce qui est ennuyeux et, donc, pas très pratique pour l'utilisateur.

La nouvelle mise en oeuvre décrite est caractérisée en ce qu'elle dispose comme supplément, d'une lame métallique élastique, à surface externe grenelée, telle qu'une lime, qui pourra être placée aux fenêtre où auparavant il y avait la lame tranchante, tout cela étant possible car ces fenêtres, qui sont généralement parallèles entre elles, mais obliques par rapport à l'axe du propre dispositif, dans la mise en oeuvre qui est maintenant décrite sont parallèles entre elles mais perpendiculaires audit axe, et donc la lame supplémentaire pourra avantageusement être placée immédiatement d'une façon sûre et rapide.

Le supplément qui a été cité reste ainsi placé, maintenu à sa place para sa propre élasticité et en offrant à l'extérieur une surface convexe munie d'une série d'emboutissages, ouverts en partie à leurs extrémités, qui constituent une surface grenelée à arêtes vives, semblables à celles de la surface d'une lime, et qui, lorsque l'on la frotte sur la surface endurcie de la peau, dans la zone connue comme durillons, produira un effet de raclage, en rabaissant ces couches de peau endurcie, formées par des cellules épithéliales mortes et en éliminant ainsi les excès, le soulagement souhaité étant ainsi obtenu.

Pour mieux comprendre la présente description, une feuille de dessins est annexée dans laquelle, à titre d'exemple non limitatif, un cas pratique du dispositif pour le soin et le traitement a été représenté, ayant les caractéristiques générales énoncées.

Dans ces dessins,

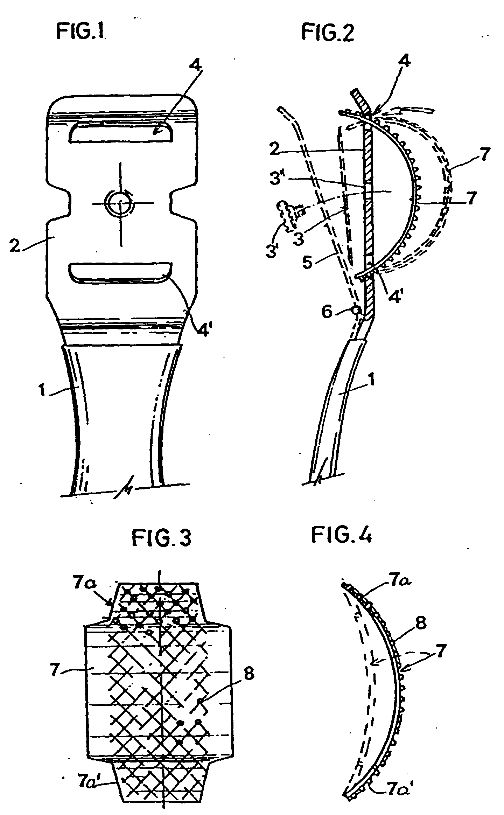

La figure 1 est une vue de front de la partie supérieure du dispositif cité;
La figure 2 est une vue de côté partiellement en coupe, correspondant à la figure précédente.
La figure 3 est une vue de front de la lame grenelée; et
La figure 4 est une vue de côté de cette lame, correspondant à la figure précédente.

Le dispositif objet de cette demande est constitué par un manche (1) en matériau, aux formes et dimensions variables, à l'extrémité supérieure duquel reste fixée la cuiller-support (2) sur laquelle sera située, de façon déjà connue, la lame (3) pour l'action tranchante dont on aura besoin, cette lame restant fixée lorsque ses extrémités tranchantes supérieure et inférieure sont introduites dans les fenêtres parallèles (4)(4') que possède la cuiller-support (2) à ses zones haute et basse, respectivement.

Ces deux fenêtres (4) (4') restent situées symétriquement suivant les axes de la cuiller-support (2) et perpendiculaires à l'axe longitudinal vertical de l'ensemble.

Le support basculant (5), mobile et tournant sur une charnière (6) située à son pied, aide activement, et il permettra de retenir et de fixer la lame tranchante (3), tout cela dans une mise en oeuvre connue et toujours avec l'aide d'une vis (3') éventuelle traversant cette lame (3) et se vissant sur l'orifice (3'') percé dans la cuiller-support (2).

Le dispositif est complété d'une façon nouvelle par une lame métallique et élastique (7) à forme généralement quadrangulaire avec ses deux côtés opposés, d'extrémité, un peut en retrait et à forme trapézoïdale (7a) (7a'). Cette lame flexible a son profilé courbé, comme on voit dans les dessins, et c'est cette forme qu'elle conservera après avoir été placée à sa place d'usage, sur la cuiller-support (2), ses extrémités respectives (7a) (7a') étant emboîtées par la flexion de la propre lame (7) à l'intérieur des fenêtres respectives (4) (4'), et présentant vers le dehors, dans leur convexité, un ensemble de grains (8), formés par emboutissage de la propre lame et ultérieur semi-découpage à l'emporte-pièce de ses extrémités, de sorte que ces grains présenteront des arêtes tranchantes, nécessaires pour réaliser l'opération de raclage requise.

Ces grains (8) sont normalement situés de façon régulière et distribués sur toute la surface de la lame (7).

Les particularités du dispositif objet de la présente invention sont essentiellement précisées dans le fait que, tel comme il a été décrit et conformément à ce qui est représenté dans les diverses figures, par cette lame complémentaire et caractéristique (7), qui est détachable, facile à placer, ce qui permet une utilité maximale du support de base (1) et (2), dans ses diverses fonctions de raclage et de coupage, comme il conviendra.

Cette opération de pose et de dépose se fera manuellement en pressant les extrémités (7a) et (7a') avec les doigts en vainquant la flexibilité propre à la lame (7), en pouvant donc introduire les deux extrémités dans les fenêtres respectives (4) (4'), et de la cuiller-support (2) ladite lame (7) restant donc parfaitement fixée lorsqu'elle va récupérer la courbe initiale. Cette position sera conservée fixe et inaltérable pendant que l'opération de raclage aura lieu.

Il faut indiquer que les formes, les dimensions et la finition ainsi que les matériaux employés dans la mise en oeuvre du dispositif qui a été décrit seront indépendants de l'objet de cette invention, pourvu que ces variations n'en altèrent pas l'esprit.

## Revendications

1. Dispositif pour le soin et le traitement de cors et de durillons constitué par un manche de base à l'extrémité duquel est agencée une pièce métallique comme cuiller-support pourvue du dispositif basculant de tenue et de fixation de la lame utilisée pour l'opération de coupage, caractérisé en ce que cette cuiller-support (2) dispose de deux fenêtres (4) (4') rectangulaires, parallèles entre elles et perpendiculaires à l'axe de l'ensemble, utilisées pour fixer la lame (3) et à travers desquelles apparaissent ses bords tranchants, tout cela de sorte qu'on pourra y placer et fixer à volonté une lame complémentaire (7), métallique et élastique, à forme courbe qui sera fixée par flexion dans ces fenêtres (4) (4'), la convexité de cette lame restant vers l'extérieur.

2. Dispositif pour le soin et le traitement de cors et de durillons, conformément à la revendication précédente, caractérisé en ce que cette lame complémentaire (7) à profilé et forme convexe, possède une série de grains (8), formés par emboutissage et complétés par découpage partiel à l'emporte-pièce, de sorte que tous présentent des arêtes d'extrémité tranchantes, pour exercer la fonction de raclage requise, étant par ailleurs caractérisé en ce que lesdits grains occupent toute la surface externe de la lame et sont disposés, de préférence, de façon régulière.
